# EUROPEAN PATENT APPLICATION

(11) **EP 3 791 877 A1**
(43) Date of publication of application: **17.03.2021**
(21) Application number: 19800673.6
(22) Date of filing: 09.05.2019
(51) Int. Cl.: A61K 31/4453, A61P 13/08, A61P 35/00, A61P 35/04

(54) **PROPHYLACTIC OR THERAPEUTIC AGENT FOR PROSTATE CANCER**

(30) Priority: 10.05.2018 JP 2018091237
(71) Applicant: Keio University, Tokyo 108-8345 (JP)
(72) Inventor: KOSAKA, Takeo, Tokyo 160-8582 (JP); HONGO, Hiroshi, Tokyo 160-8582 (JP); OYA, Mototsugu, Tokyo 160-8582 (JP)
(74) Representative: Script IP Limited
(86) International application number: PCT/JP2019/018465
(87) International publication number: WO 2019/216360

(57) **Abstract**

The present invention provides a prophylactic or therapeutic agent for prostate cancer which can be produced relatively conveniently and inexpensively and which contains as an active ingredient a low-molecular compound that exhibits an anticancer effect even on castration-resistant prostate cancer which is refractory (in particular, castration-resistant prostate cancer with cabazitaxel resistance, which is the most refractory prostate cancer). Cloperastine or a pharmaceutically acceptable salt thereof is used as the prophylactic or therapeutic agent for prostate cancer (preferably castration-resistant prostate cancer with cabazitaxel resistance).

## Description

### Technical Field

The present invention relates to a preparation for prevention and/or treatment of prostate cancer, which comprises cloperastine or a pharmaceutically acceptable salt thereof (hereinafter, sometimes referred to collectively as a "cloperastine species").

### Background Art

Prostate cancer is a cancer that occurs in the prostate which is one of male genitalia, and in the United States and European nations, particularly in the United States, prostate cancer is the most common cancer in the male population. It is considered that prostate cancer is linked to eating habits with high intakes of animal fat, and advanced age. In fact, in Japan, the number of patients with prostate cancer has been rapidly increasing in recent years due to westernization of eating habits and aging of population, and for example, prostate cancer was the fourth in terms of the number of patients among cancers classified by site in 2013. The morbidity rate of prostate cancer dramatically increases after the age of 70, and is therefore predicted to further rise in developed countries including Japan, where the population is aging. Accordingly, development of an effective method for treatment of prostate cancer is required.

Clinically, surgical therapy, hormone therapy, chemotherapy, radiation therapy and the like are known as methods for treatment of prostate cancer. Of these, surgical therapy such as surgery to remove the prostate is a first-line choice, and when a diagnosis is made as an advancing cancer, or it is difficult to undergo an operation due to recurrence after surgical therapy, a treatment method such as hormone therapy, or chemotherapy with an anticancer agent such as docetaxel is selected.

Growth of prostate cancer is stimulated by androgen which is a male hormone, and an androgen receptor (AR) is known to play a major role in occurrence and progression of the cancer. Thus, a method for suppressing the action of androgen (androgen deprivation therapy (ADT)) is common as hormone therapy (endocrine therapy) for prostate cancer. ADT is effective on a temporary basis, and normally, within several years, the prostate cancer develops into hormone refractory prostate cancer (HRPC) and androgen-independent prostate cancer (AIPC) having resistance to ADT. For castration-resistant prostate cancer (CRPC) (also referred to as "recurrent progressive prostate cancer"), docetaxel and enzalutamide are used, and cancer cells having resistance to these drugs have emerged, which poses a clinical problem. As one of means for countering such a problem, a treatment method which is used in combination with docetaxel or the like has been reported (Patent Document 1).

New taxane-based anticancer agents applicable to prostate cancer include cabazitaxel. Cabazitaxel has been confirmed to exhibit an antitumor effect even on castration-resistant prostate cancer with a history of pretreatment involving docetaxel, and should be called the last bastion for medical therapy. Cabazitaxel is approved for an indication for the treatment of hormone treatment-resistant prostate cancer in the United States and European nations, and was approved for an indication for the treatment of prostate cancer in Japan on July 4, 2014. It has been considered that since cabazitaxel has low affinity with P-glycoprotein (P-gp), drug resistance to cabazitaxel due to P-gp activation is less likely to occur although cabazitaxel is similar in structure to paclitaxel and docetaxel. However, cases of cabazitaxel-resistant prostate cancer have been reported, and treatment of cabazitaxel-resistant prostate cancer is very imperative.

On the other hand, Hustazol (Nipro ES Pharma co., Ltd.) containing cloperastine hydrochloride as an active ingredient is commercially available as an antitussive agent. However, it has not been heretofore known that cloperastine has a prophylactic or therapeutic effect on prostate cancer.

### Prior Art Document

### Patent Document

Patent Document 1: Japanese Patent Publication No. 5605511

### Summary of the Invention

### Object to be Solved by the Invention

An object of the present invention is to provide a prophylactic or therapeutic agent for prostate cancer which can be produced relatively conveniently and inexpensively and which contains as an active ingredient a low-molecular compound that exhibits an anticancer effect even on castration-resistant prostate cancer which is refractory (in particular, castration-resistant prostate cancer with cabazitaxel resistance, which is the most refractory prostate cancer).

### Means to Solve the Object

For CRPC patients who had acquired cabazitaxel resistance through CRPC treatment with a new AR inhibitor, a formalin fixed paraffin embedded (FFPE) sample of CRPC tissues before the treatment (before acquirement of cabazitaxel resistance) and a FFPE sample of CRPC tissues after the treatment (after acquirement of cabazitaxel resistance) were used to prepare gene expression profiles in the respective samples. Compounds capable of changing the gene expression profile in CRPC after acquirement of cabazitaxel resistance to the gene expression profile in CRPC before acquirement of cabazitaxel resistance are then analyzed, and resultantly, 70 kinds of compounds were obtained as candidate drugs for prostate cancer as a result of screening. For these 70 kinds of candidate drugs for prostate cancer, anticancer effects on metastatic CRPC cell lines and cabazitaxel-resistant CRPC cell lines were examined. While there were many compounds having no anticancer effect, it was found after many trials that cloperastine had a high anticancer effect on metastatic CRPC and cabazitaxel-resistant CRPC cell lines. In addition, it was confirmed that when used in combination with an existing anticancer agent (cabazitaxel), cloperastine further enhanced an anticancer effect on cabazitaxel-resistant prostate cancer. The present invention has been completed on the basis of these findings.

That is, the present invention is as follows.
[1] A prophylactic or therapeutic agent for prostate cancer, comprising cloperastine or a pharmaceutically acceptable salt thereof.
[2] The prophylactic or therapeutic agent according to above [1], which is orally administered.
[3] The prophylactic or therapeutic agent according to above [1] or [2], wherein the prostate cancer is castration-resistant prostate cancer or enzalutamide-resistant prostate cancer.
[4] The prophylactic or therapeutic agent according to above [3], wherein the castration-resistant prostate cancer has cabazitaxel resistance.
[5] The prophylactic or therapeutic agent according to any one of above [1] to [4], which is used in combination with an anticancer agent.
[6] The prophylactic or therapeutic agent according to above [5], wherein the anticancer agent is cabazitaxel.

Other embodiments of the present invention include a method for preventing or treating prostate cancer, comprising the step of administering a cloperastine species to a subject in need of prevention or treatment of prostate cancer; a cloperastine species which is used as a prophylactic or therapeutic agent for prostate cancer; a cloperastine species which is used in prevention or treatment of prostate cancer; and use of a cloperastine species for producing a prophylactic or therapeutic agent for prostate cancer.

### Effect of the Invention

The cloperastine species exhibits an excellent anticancer (antitumor) effect on prostate cancer, particularly enzalutamide-resistant prostate cancer or refractory CRPC, in particular cabazitaxel-resistant CRPC which is the most refractory prostate cancer, by actions such as suppressive action on growth of prostate cancer cells, suppressive action on epithelial mesenchymal transition (EMT) in prostate cancer cells, suppressive action on invasion of prostate cancer cells and apoptosis inducing action on prostate cancer cells. Such an effect can be further enhanced by using the cloperastine species in combination with an existing anticancer agent (preferably cabazitaxel). In addition, the prophylactic or therapeutic agent for prostate cancer according to the present invention is advantageous in that it can be produced relatively conveniently and inexpensively because the cloperastine species which is a low-molecular compound that can be relatively conveniently produced is used as an active ingredient for prevention or treatment of prostate cancer.

### Brief Description of Drawings

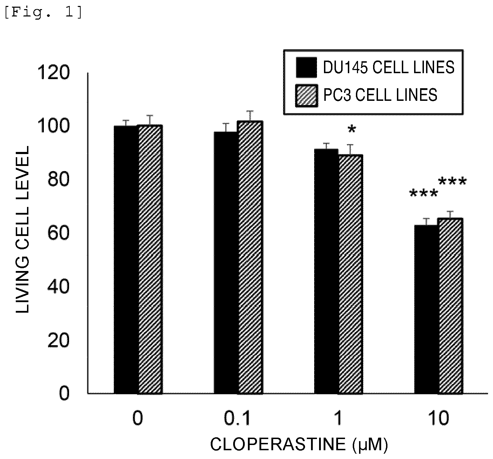

[Figure 1] Figure 1 shows the results of analyzing living cell levels in culturing of DU145 cell lines and PC3 cell lines in the presence of cloperastine at various concentrations (0 µM, 0.1 µM, 1 µM and 10 µM). The "living cell level" on the ordinate is shown as a relative value (average value + standard deviation [SD]) against the number of living cells in culturing without cloperastine, which is defined as 100. "*" and "***" in the figure indicate that there is a statistically significant difference between the results of culturing in the presence of cloperastine and without cloperastine (0 µM) (p < 0.05 and p < 0.001, respectively).

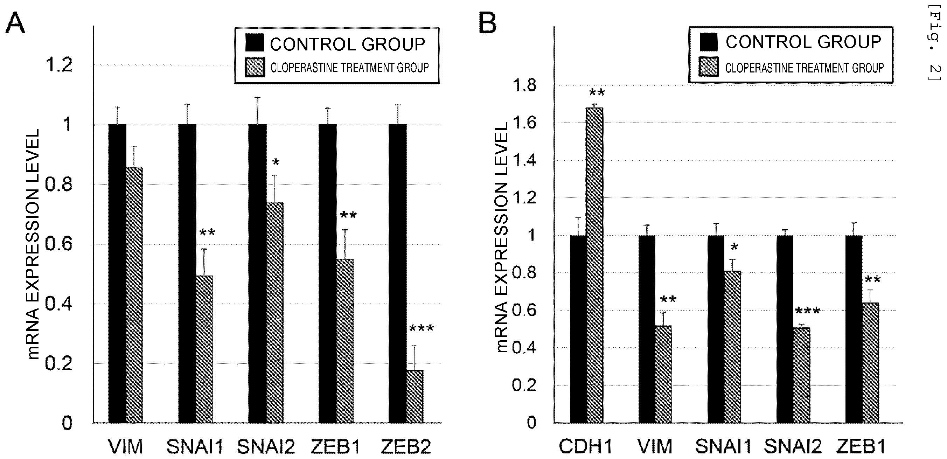

[Figure 2] Figure 2 shows the results of analyzing mRNA expression levels of six genes (CDH1 gene, VIM gene, SNAI1 gene, SNAI2 gene, ZEB1 gene and ZEB2 gene) in culturing of DU145 cell lines (Figure 2A) and PC3 cell lines (Figure 2B) in the presence of cloperastine (cloperastine treatment group) or without cloperastine (control group). The "mRNA expression level" on the ordinate is shown as a relative value (average value + standard deviation [SD]) against the expression level in the control group, which is defined as 1, for each of the genes. "*", "**" and "***" in the figure indicate that there is a statistically significant difference between the cloperastine treatment group and the control group (p < 0.05, p < 0.01 and p < 0.001, respectively).

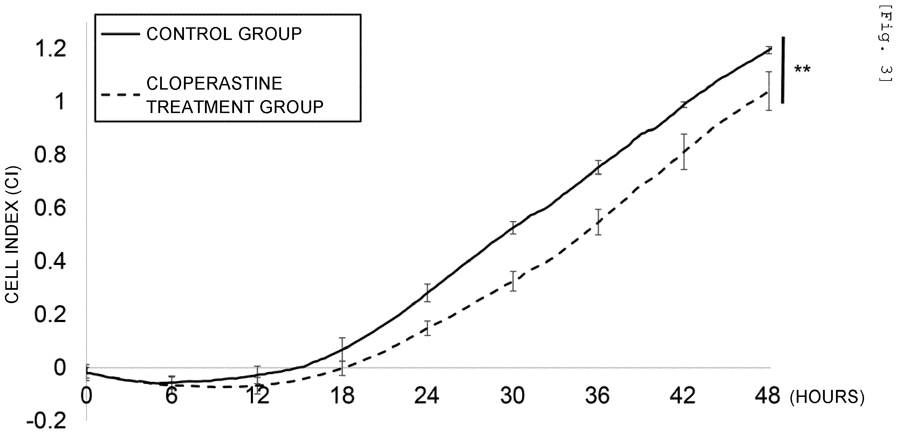

[Figure 3] Figure 3 shows the results of analyzing cell invasiveness in culturing of DU145 cell lines in the presence of cloperastine (cloperastine treatment group) or without cloperastine (control group) (average value ± standard deviation [SD]).

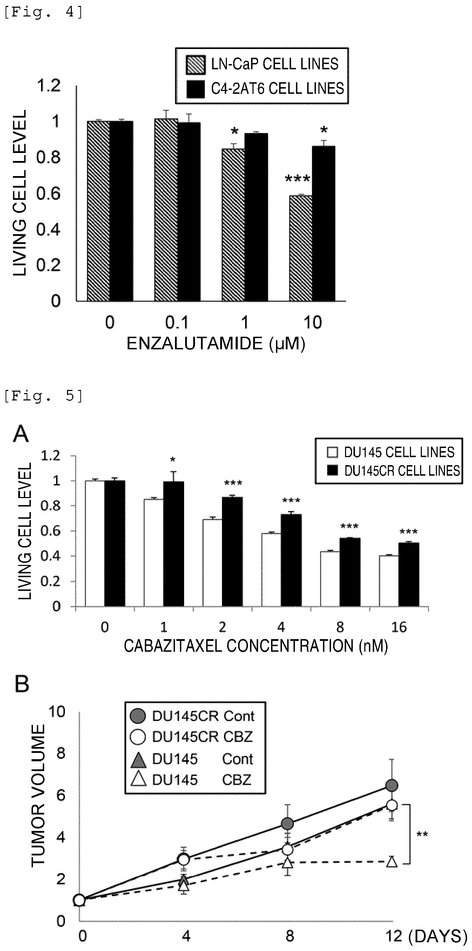

[Figure 4] Figure 4 shows the results of analyzing living cell levels in culturing of LN-CaP cell lines and C4-2AT6 cell lines in the presence of enzalutamide at various concentrations (0 µM, 0.1 µM, 1 µM and 10 µM). The "living cell level" on the ordinate is shown as a relative value (average value + standard deviation [SD]) against the number of living cells in culturing without enzalutamide, which is defined as 1. "*" and "***" in the figure indicate that there is a statistically significant difference between the results of culturing in the presence of enzalutamide and without enzalutamide (0 µM) (p < 0.05 and p < 0.001, respectively).
[Figure 5] Figure 5A shows the results of analyzing living cell levels *in vitro* in culturing of DU145 cell lines and DU145CR cell lines in the presence of cabazitaxel at various concentrations (0 nM, 1 nM, 2 nM, 4 nM, 8 nM and 16 nM) . The "living cell level" on the ordinate is shown as a relative value (average value + standard deviation [SD]) against the number of living cells in culturing without cabazitaxel, which is defined as 1. "*" and "***" in the figure indicate that there is a statistically significant difference between the DU145 cell line and the DU145CR cell line (p < 0.05 and p < 0.001, respectively). Figure 5B shows the results of analyzing a change in tumor volume for two groups of subcutaneous tumor model mice prepared with DU145 cell lines (DU145 mouse group [DU145 Cont] and cabazitaxel administration DU145 mouse group [DU145 CBZ]) and two groups of subcutaneous tumor model mice prepared with DU145CR cell lines (DU145CR mouse group [DU145CR Cont] and cabazitaxel administration DU145CR mouse group [DU145CR CBZ]). The "tumor volume" on the ordinate is shown as a relative value (average value ± standard deviation [SD]) against the tumor volume in the DU145 mouse group or the DU145CR mouse group immediately after administration (day 0), which is defined as 1. The "number of days" on the abscissa shows the number of days after administration of cabazitaxel. "**" in the figure indicates that there is a statistically significant difference between the cabazitaxel administration DU145CR mouse group and the cabazitaxel administration DU145 mouse group (p < 0.01).

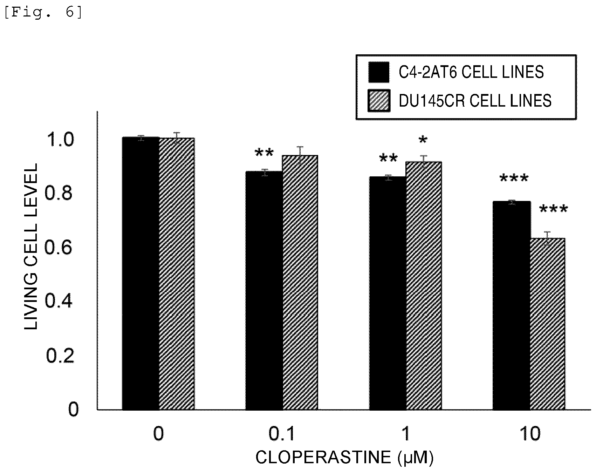

[Figure 6] Figure 6 shows the results of analyzing living cell levels in culturing of C4-2AT6 cell lines and DU145CR cell lines in the presence of cloperastine at various concentrations (0 µM, 0.1 µM, 1 µM and 10 µM). The "living cell level" on the ordinate is shown as a relative value (average value ± standard deviation [SD]) against the number of living cells in culturing without cloperastine (0 µM), which is defined as 1. "*", "**" and "***" indicate that there is a statistically significant difference between the results of culturing in the presence of cloperastine and without cloperastine (p < 0.05, p < 0.01 and p < 0.001, respectively).

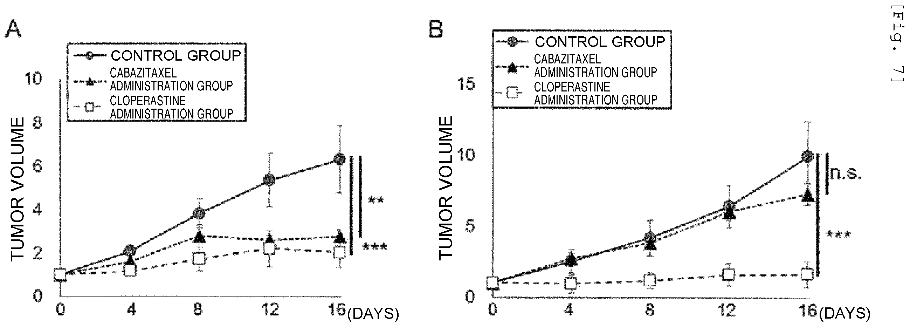

[Figure 7] Figure 7A shows the results of analyzing a change in tumor volume for three groups of subcutaneous tumor model mice prepared with DU145 cell lines (control group, cabazitaxel administration group and cloperastine administration group). Figure 7B shows the results of analyzing a change in tumor volume for three groups of subcutaneous tumor model mice prepared with DU145CR cell lines (control group, cabazitaxel administration group and cloperastine administration group). The "tumor volume" on the ordinate is shown as a relative value (average value ± standard deviation [SD]) against the tumor volume in the control group immediately after administration (day 0), which is defined as 1. The "number of days" on the abscissa shows the number of days after administration of cabazitaxel or cloperastine. "n.s." in Figure 7B indicates that there is no statistically significant difference between the cabazitaxel administration group and the control group on day 16 after administration (p > 0.05). "**" in Figure 7A indicates that there is a statistically significant difference between the cabazitaxel administration group and the control group on day 16 after administration (p < 0.01). "***" in Figures 7A and 7B indicates that there is a statistically significant difference between the cloperastine administration group and the control group on day 16 after administration (p < 0.001).

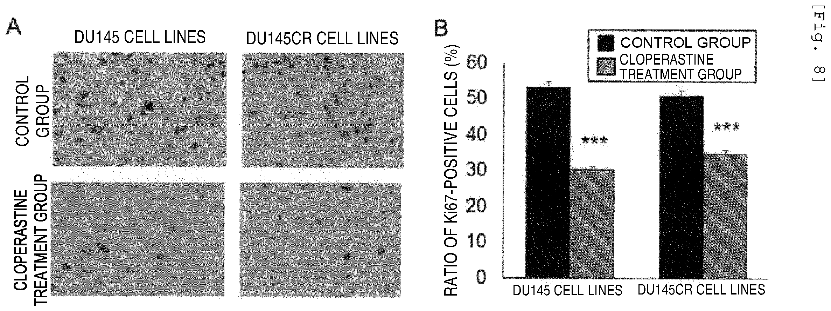

[Figure 8] Figure 8 shows the results of analyzing ratios of Ki67 expression cells in culturing of DU145 cell lines and DU145CR cell lines in the presence of cloperastine (cloperastine treatment group) or without cloperastine (control group). "***" in the figure indicates that there is a statistically significant difference between the cloperastine treatment group and the control group (p < 0.001) .

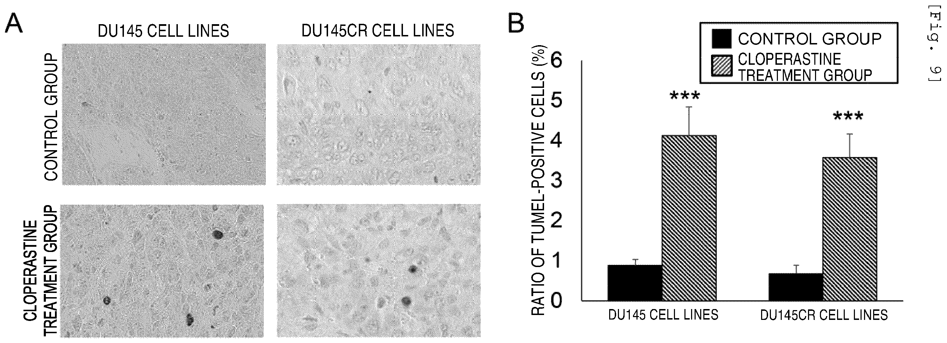

[Figure 9] Figure 9 shows the results of analyzing ratios of TUNEL-positive cells in culturing of DU145 cell lines and DU145CR cell lines in the presence of cloperastine (cloperastine treatment group) or without cloperastine (control group). "***" in the figure indicates that there is a statistically significant difference between the cloperastine treatment group and the control group (p < 0.001) .

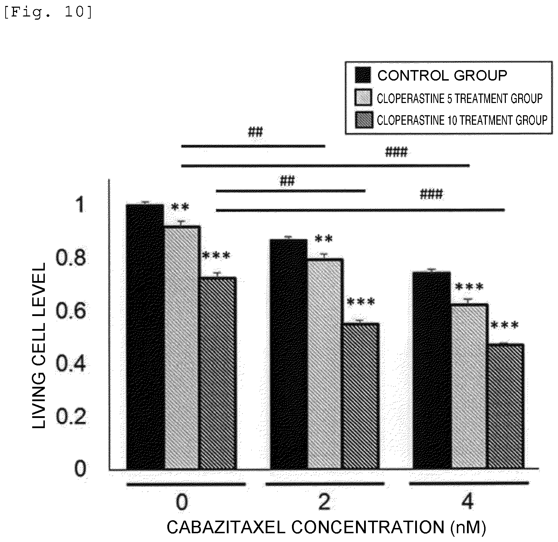

[Figure 10] Figure 10 shows the results of analyzing living cell levels in culturing of DU145CR cell lines in the presence of 2 nM cabazitaxel, in the presence of 4 nM cabazitaxel or without cabazitaxel (0 nM) and in the presence of 5 µM cloperastine (cloperastine 5 treatment group), in the presence of 10 µM cloperastine (cloperastine 10 treatment group) or without cloperastine (control group) . The "living cell level" on the ordinate is shown as a relative value (average value + standard deviation [SD]) against the number of living cells in the control group without cabazitaxel, which is defined as 1. "**" and "***" in the figure indicate that there is a statistically significant difference between each of the cloperastine treatment groups and the control group at each concentration of cabazitaxel (p < 0.01 and p < 0.001, respectively). "##" and "###" in the figure indicate that there is a statistically significant difference between the results in the presence of cabazitaxel and without cabazitaxel in the cloperastine 5 treatment group and the cloperastine 10 treatment group (p < 0.01 and p < 0.001, respectively).

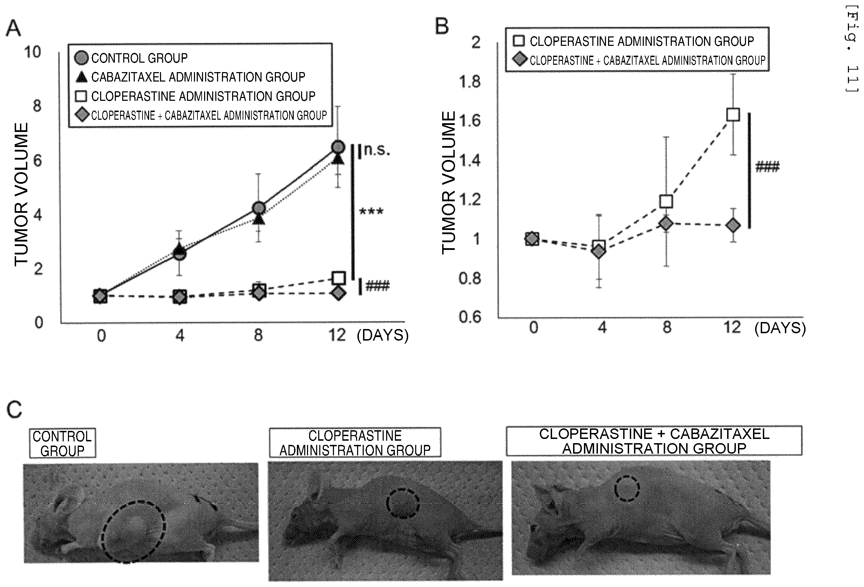

[Figure 11] Figure 11A shows the results of analyzing a change in volume of the tumor (area surrounded by dotted line in Figure 11C) for four groups of subcutaneous tumor model mice prepared with DU145CR cell lines (control group, cabazitaxel administration group, cloperastine administration group and cloperastine + cabazitaxel administration group). The "tumor volume" on the ordinate is shown as a relative value (average value ± standard deviation [SD]) against the tumor volume in the control group immediately after administration (day 0), which is defined as 1. The "number of days" on the abscissa shows the number of days after administration. Figure 11B are diagrams obtained by extracting and enlarging the results for the cloperastine administration group and the cloperastine + cabazitaxel administration group in Figure 11A. "n.s." in Figure 11A indicates that there is no statistically significant difference between the cabazitaxel administration group and the control group on day 12 after administration (p > 0.05). "***" in Figures 11A and 11B indicates that there is a statistically significant difference between the cloperastine administration group and the control group on day 12 after administration (p < 0.001). "###" in Figures 11A and 11B indicates that there is a statistically significant difference between the cloperastine + cabazitaxel administration group and the cloperastine administration group on day 12 after administration (p < 0.001).

### Mode of Carrying Out the Invention

The prophylactic or therapeutic agent for prostate cancer according to the present invention is a preparation comprising a cloperastine species, whose use is limited to "prevention or treatment of prostate cancer" (hereinafter, sometimes referred to as "the present prophylactic/therapeutic agent"). Here, the prevention of prostate cancer includes prevention of occurrence of prostate cancer, and prevention of worsening of symptoms of prostate cancer. The present prophylactic/therapeutic agent may be used alone as a medicament (preparation), or may be mixed with additives, and used as a composition form (pharmaceutical composition).

The prostate cancer to be prevented or treated may be cancer in which the organ developing a malignant tumor (cancer) is a prostate, and examples of the cancer include localized, invasive or metastatic prostate cancer; castration-resistant, AR-targeting drug (e.g. enzalutamide or abiraterone)-resistant, taxane-based anticancer agent (e.g. paclitaxel, docetaxel or cabazitaxel)-resistant, or castration-resistant and AR-targeting drug-resistant prostate cancer, castration-resistant and taxane-based anticancer agent-resistant prostate cancer, or castration-resistant, AR-targeting drug-resistant and taxane-based anticancer agent-resistant prostate cancer; localized, invasive or metastatic and castration-resistant prostate cancer; localized, invasive or metastatic and AR-targeting drug-resistant prostate cancer; localized, invasive or metastatic and taxane-based anticancer agent-resistant prostate cancer; localized, invasive or metastatic, castration-resistant and taxane-based anticancer agent-resistant prostate cancer; and localized, invasive or metastatic, castration-resistant, AR-targeting drug-resistant and taxane-based anticancer agent-resistant prostate cancer. The prostate cancer is preferably prostate cancer with at least castration resistance, prostate cancer with at least enzalutamide resistance, or prostate cancer with at least cabazitaxel resistance, more preferably prostate cancer with at least castration resistance and cabazitaxel resistance because the effect of the prophylactic or therapeutic agent on such cancer has been demonstrated as described in Examples below.

In the present description, the "localized prostate cancer" means non-filtrating and non-metastasizing prostate cancer, i.e. a state in which the cancer does not spread to tissues or organs other than the prostate, and remains within the prostate. This cancer corresponds to stage A or B prostate cancer. In the present description, the "invasive prostate cancer" means a state in which the cancer invades tissues and organs around the prostate (e.g. bladder, rectum or seminal vesicle), and does not metastasize. This cancer corresponds to stage C prostate cancer. In the present description, the "metastatic prostate cancer" means a state in which the cancer metastasizes, i.e. the cancer enters blood vessels and lymphatic vessels, and spreads to tissues or organs away from the prostate (e.g. lymphatic node, bone [e.g. spinal column or pelvic bone], liver or lung) along with the flow of blood and lymphatic fluid. This cancer corresponds to stage D prostate cancer.

In the present description, the "castration-resistant prostate cancer (CRPC)" means prostate cancer in which the disease is exacerbated or the prostate specific antigen (PSA) level rises with no relation to administration of an anti-androgenic agent although surgical castration or chemical castration has been provided, and the serum testosterone is less than 50 ng/dL. That is, CRPC means a state in which although secretion of androgen from the testicle is eliminated, so that the concentration of androgen in the blood is very low, prostate cancer progresses, and the PSA value rises.

The "invasive prostate cancer" to be prevented or treated includes cancer in the prostate, and cancer in tissues or organs invaded by the prostate cancer for convenience, and cancer in the prostate is preferable. The "metastatic prostate cancer" to be prevented or treated includes cancer in the prostate, and cancer in tissues or organs, to which the prostate cancer metastasizes, for convenience, and cancer in the prostate is preferable.

In the present description, the "cabazitaxel-resistant prostate cancer" means prostate cancer having resistance to cabazitaxel, more specifically prostate cancer in which in an *in vivo* case, the tumor volume increases when cabazitaxel is administered to a prostate cancer patient for at least 14 days at 10 mg/kg (body weight) per day. In an *in vitro* case, the resistance to cabazitaxel of cabazitaxel-resistant prostate cancer cell lines is at least 1.2 times or more, preferably 1.5 times or more, more preferably 2.0 times or more, still more preferably 2.3 times or more, furthermore preferably 2.6 times or more, most preferably 2.8 times or more of that of cabazitaxel non-resistant prostate cancer cell lines in terms of 50% inhibition concentration (IC50).

In the present description, the "enzalutamide-resistant prostate cancer" means prostate cancer having resistance to enzalutamide, more specifically prostate cancer in which in an *in vivo* case, the tumor volume increases when enzalutamide is administered to a prostate cancer patient for at least 14 days at 25 mg/kg (body weight) per day. In an *in vitro* case, the resistance to enzalutamide of enzalutamide-resistant prostate cancer cell lines is at least 1.5 times or more, preferably 2.0 times or more, more preferably 4.0 times or more, still more preferably 5.0 times or more, furthermore preferably 7.0 times or more, most preferably 7.7 times or more of that of enzalutamide non-resistant prostate cancer cell lines in terms of 50% inhibition concentration (IC50).

The cloperastine according to the present invention is 1-{2-[(RS)-(4-chlorophenyl)(phenyl)methoxy]ethyl}piperidine, and is a compound represented by the following formula.

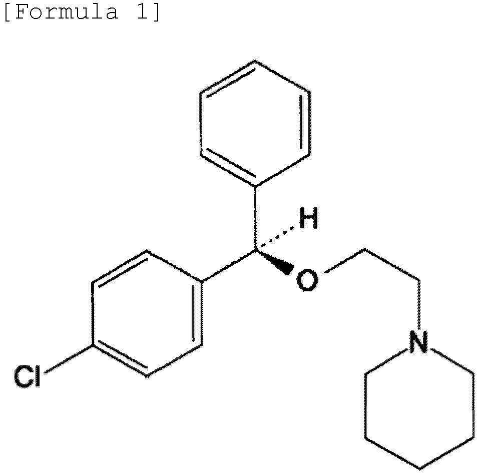

The pharmaceutically acceptable salt of cloperastine in the cloperastine species may be pharmaceutically acceptable, and examples thereof include an acid addition salt such as a hydrochloride, a hydrobromide, a hydroiodide, a phosphate, a nitrate, a sulfate, an acetate, a propionate, a toluenesulfonate, a succinate, an oxalate, a lactate, a tartrate, a glycolate, a methanesulfonate, a butyrate, a valerate, a citrate, a fumarate, a maleate, a malate or a fendizoate.

Preferably, an anticancer agent is used in combination with the present prophylactic/therapeutic agent for further enhancing the anticancer action on prostate cancer. The anticancer agent used in combination with the present prophylactic/therapeutic agent may be a substance known to suppress growth of cancer (preferably growth of prostate cancer) by action such as inhibition of DNA replication or inhibition of cell division, and examples of the anticancer agent include cyclophosphamide, dacarbazine, chlorambucil, methotrexate, cytarabine, actinomycin D, bleomycin, doxorubicin, vincristine, vinblastine, cisplatin, oxaliplatin, carboplatin, irinotecan, streptozocin, paclitaxel, docetaxel, cabazitaxel, etoposide, gemcitabine, bevacizumab, rituximab, brefeldin A, trastuzumab, imatinib, pemetrexed, capecitabine, bortezomib, leuprorelin, erlotinib, sunitinib, cetuximab, goserelin, dasatinib, sorafenib, 5-fluorouracil (FU), enzalutamide and abiraterone. Of these, cabazitaxel can be mentioned as a preferred example because the effect of cabazitaxel used in combination with the present prophylactic/therapeutic agent has been demonstrated as described in Examples below.

The present prophylactic/therapeutic agent may further contain additives such as a pharmacologically acceptable normal carrier, binder, stabilizer, excipient, diluent, pH buffer, disintegrant, isotonic agent, additive, covering agent, solubilizer, wetting agent, solubilizing agent, lubricant, flavoring agent, sweetening agent, solvent, gelling agent and nutrient. Specific examples of the additives include water, physiological saline, animal fat and oil, plant oil, lactose, starch, gelatin, crystalline cellulose, gum, talc, magnesium stearate, hydroxypropylcellulose, polyalkylene glycol, polyvinyl alcohol and glycerin.

Examples of the administration form of the present prophylactic/therapeutic agent include oral administration in which a dosage form such as a powder, a granule, a tablet, a capsule, a syrup or a suspension is administered; and parenteral administration in which a dosage form such as a solution, an emulsion or a suspension is injected (e.g. subcutaneously, intravenously or intramuscularly injected), or intranasally administered in the form of a spray, and oral administration is preferable.

The dose of the cloperastine species in the present prophylactic/therapeutic agent is appropriately determined according to an age, a body weight, a sex, a symptom, sensitivity to the drug, and the like, and is, for example, in the range of 0.1 µg to 200 mg/kg (body weight) per day. In Examples described below, experiments with model mice indicate a dose of 12.5 mg/kg per day as a specific dose of cloperastine. Conversion of the dose into a dose to a human on the basis of a human equivalent dose (HED) in mouse of 12.3 (see literature "Guidance for Industry Estimating the Maximum Safe Starting Dose in Initial Clinical Trials for therapeutics in Adult Healthy Volunteers") gives a dose of about 1.02 mg/kg per day. Thus, the dose of cloperastine species in the present prophylactic/therapeutic agent is preferably 1 µg to 100 mg/kg per day, more preferably 10 µg to 50 mg/kg per day, still more preferably 30 µg to 15 mg/kg per day, furthermore preferably 100 µg to 8 mg/kg per day, most preferably 500 µg to 1.5 mg/kg per day. The present prophylactic/therapeutic agent may be administered in a single dose or in two or more (e.g. 2 to 4) divided doses per day.

The present prophylactic/therapeutic agent may contain an ingredient having anticancer action on prostate cancer, in addition to the cloperastine species, or may be free of an anti-prostate cancer action ingredient other than the cloperastine species (e.g. protein, DNA, RNA, plant-derived extracts or polymer) because even cloperastine alone exhibits anti-prostate cancer action (e.g. suppressive action on growth of prostate cancer cells, suppressive action on EMT in prostate cancer cells, suppressive action on invasion of prostate cancer cells or apoptosis inducing action on prostate cancer cells).

The cloperastine species can be produced by any of known methods such as chemical synthesis, production with microorganisms and production with enzyme, and a commercialized product can be used. Examples thereof include a cloperastine hydrochloride (Hustazol [registered trademark] Sugar-Coated Tablets 10 mg manufactured by Nipro ES Pharma co., Ltd.), a cloperastine hydrochloride (manufactured by Sigma-Aldrich Co. LLC), and a cloperastine fendizoate (HUSTAZOL [registered trademark] Powder 10% manufactured by Nipro ES Pharma co., Ltd.).

Hereinafter, the present invention will be described in more detail by way of Example, which should not be construed as limiting the technical scope of the present invention. In Examples below, various cell lines were cultured at 37°C at 5% CO₂/20% O₂ in an RPMI1640 culture solution.

### Example 1

### 1. Screening of Candidate Drugs for Prostate Cancer

For CRPC patients (n = 3) who had acquired cabazitaxel resistance through CRPC treatment with a new AR inhibitor, mRNA was extracted with "RNeasy DSP FFPE kit" (manufactured by QIAGEN Company) from an FFPE sample of CRPC tissues before the treatment (before acquirement of cabazitaxel resistance) and an FFPE sample of CRPC tissues after the treatment (after acquirement of cabazitaxel resistance) under the approval of Keio University Ethics Committee. The extracted RNA was subjected to fragmentation into cDNA and labeling with "Affymetrix GeneChip WT Terminal Labeling Kit" (manufactured by Affymetrix Company), and washed with "GeneChip Fluidics Station 450" (manufactured by Affymetrix Company), and microarray analysis was performed with a microarray system (GeneChip Scanner 3000 7G manufactured by Affymetrix Company) to analyze the ratio of change in gene expression profile from CRPC cells after acquirement of cabazitaxel resistance to CRPC cells before acquirement of cabazitaxel resistance. CMAP analysis from Broad Institute, USA was then applied to compare and study the above-mentioned ratio of change and a ratio of change in gene expression profile based on a library of clinically usable compounds, and 70 kinds of compounds capable of changing the gene expression profile in cabazitaxel-resistant CRPC cells to the gene expression profile in cabazitaxel non-resistant CRPC cells were found as candidate drugs for prostate cancer as a result of screening. Resultantly, it was shown that one of such candidate drugs was cloperastine. **Example 2**

### 2. Cell Growth Suppressive Effect on Prostate Cancer Cells

For confirming that the cloperastine species has a cell growth suppressive effect on metastatic CRPC, metastatic CRPC cell lines (DU145 cell lines and PC3 cell lines) were cultured in the presence of the cloperastine species, and the cell growth property was analyzed.

### [Method]

DU145 cell lines and PC3 cell lines (these cell lines were obtained from ATCC [American Type Culture Collection]) were cultured on a 96-well plate for 24 hours. Thereafter, to a culture solution was added a cloperastine hydrochloride (manufactured by Sigma-Aldrich Co. LLC) to 0.1 µM, 1 µM or 10 µM, the cell lines were further cultured for 24 hours, and the number of living cells was measured with "Premix WST-1 Cell Proliferation Assay System" (manufactured by Takara Bio Inc.). As a control, an experiment was also conducted in which DU145 cell lines and PC3 cell lines were cultured in a culture solution free of a cloperastine hydrochloride.

### [Result]

DU145 cell lines and PC3 cell lines cultured in the presence of cloperastine were shown to have a significantly smaller number of living cells in a concentration-dependent manner than the cell lines cultured without cloperastine (see Figure 1).

This result shows that the cloperastine species has a cell growth suppressive effect on metastatic CRPC.

### Example 3

### 3. EMT Suppressive Effect on Metastatic CRPC

For confirming that the cloperastine species has a suppressive effect on EMT in metastatic CRPC cells, metastatic CRPC cell lines (DU145 cell lines and PC3 cell lines) were cultured in the presence of the cloperastine species, and the gene expression profiles of EMT-related factors were analyzed.

### [Method]

DU145 cell lines and PC3 cell lines were cultured for 24 hours in the presence of 10 µM cloperastine or without cloperastine (cloperastine treatment group and control group, respectively), and mRNA was then extracted with "RNeasy Mini kit" (manufactured by QIAGEN Company). From the extracted mRNA, cDNA was synthesized with "PrimeScript RT reagent Kit" (manufactured by Takara Bio Inc.). For analyzing the mRNA expression levels of genes encoding six kinds of factors (E-cadherin, vimentin, "Zinc finger protein SNAI1", "Zinc finger protein SNAI2", "Zinc finger E-box-binding homeobox 1" and "Zinc finger E-box-binding homeobox 2") (CDH1 gene, VIM gene, SNAI1 gene, SNAI2 gene, ZEB1 gene and ZEB2 gene, respectively), quantitative PCR analysis was performed with the primer and TaqMan probe sets (TaqMan Gene Expression Assays) (manufactured by Applied Biosystems Company) shown in Table 1 below, and CFX96 Real-Time System (manufactured by Bio-Rad Laboratories, Inc).

**[Table 1]**

| Name of gene | Primer and TaqMan probe set |
|---|---|
| CDH1 gene | Hs00170423_ m1 |
| VIM gene | Hs00958111_m1 |
| SNAI1 gene | Hs00195591_m1 |
| SNAI2 gene | Hs00950344_m1 |
| ZEB1 gene | Hs01566407_m1 |
| ZEB2 gene | Hs00207691_ m1 |

### [Result]

The expression levels of mRNA encoding five kinds of proteins (vimentin, "Zinc finger protein SNAI1", "Zinc finger protein SNAI2", "Zinc finger E-box-binding homeobox 1" and "Zinc finger E-box-binding homeobox 2") related to EMT were all lower in the cloperastine treatment group than in the control group (see Figures 2A and 2B). The expression level of mRNA encoding E-cadherin lower in expression in EMT was higher in the cloperastine treatment group than in the control group (see Figure 2B).

These results indicate that the cloperastine species suppresses EMT in metastatic CRPC cells, leading to suppression of metastasis and invasion of CRPC.

### Example 4

### 4. Analysis of Invasiveness of Prostate Cancer Cells

For confirming the anti-prostate cancer effect of the cloperastine species, DU145 cell lines were cultured in the presence of the cloperastine species, and the invasiveness of the cell lines was analyzed (invasion assay).

### [Method]

To a culture solution added to each well of Real-Time Cell Analyzer xCELLigence (manufactured by ACEA Biosciences Inc.), a cloperastine hydrochloride (manufactured by Sigma-Aldrich Co. LLC) was added to 1 µM, and 4.0 × 10⁴ DU145 cell lines were added. The cell lines were then cultured, and the number of cells passing through a Matrigel basement membrane matrix (manufactured by Corning Incorporated) was measured over time (0 to 48 hours) (cloperastine treatment group). As a control for comparison, DU145 cell lines were cultured in a culture solution free of a cloperastine hydrochloride, and the same experiment was conducted (control group). Cloperastine with a concentration of 1 µM had no impact on growth of normal cells.

### [Result]

It was confirmed that when DU145 cell lines were cultured in a culture solution free of cloperastine, cells invaded (see "control group" in Figure 3). On the other hand, it was shown that when DU145 cell lines were cultured in a cloperastine-containing culture solution, invasion of cells was significantly suppressed (see "cloperastine treatment group" in Figure 3).

This results shows that the cloperastine species has a suppressive effect on invasion of prostate cancer cells, and since such an effect was exhibited even at a concentration low enough to have no impact on growth of cells, it is expected to develop a prophylactic or therapeutic agent for prostate cancer, which has little side effects.

### Example 5

### 5. Determination of Whether Androgen-Independent CRPC Model Cell Lines C4-2AT6 Has Enzalutamide Resistance

The present inventors established androgen-independent CRPC model cell lines C4-2AT6 (see literature "Kosaka et al., J Urol. 2011 Jun; 185(6): 2376-81."). Whether such C4-2AT6 cell lines had enzalutamide resistance was determined.

### [Method]

C4-2AT6 cell lines, and LN-CaP cell lines (obtained from ATCC [American Type Culture Collection]) which are androgen-dependent prostate cancer cell lines as a control for comparison were cultured on 96-well plates, respectively, for 24 hours. Thereafter, to a culture solution, enzalutamide (manufactured by Chemscene LLC) was added to various concentrations (0 µM, 0.1 µM, 1 µM and 10 µM). The cell lines were further cultured for 48 hours, and the number of living cells was then measured with "Premix WST-1 Cell Proliferation Assay System" (manufactured by Takara Bio Inc.).

### [Result]

The number of living cells in C4-2AT6 cell lines treated with enzalutamide was shown to be larger than the number of living cells in LN-CaP cell lines treated with enzalutamide (see Figure 4). On the basis of such a result, the 50% inhibition concentration (IC50) of enzalutamide was measured, and resultantly it was found that IC50 for LN-CAP cell lines was 12.1 µM, whereas IC50 for C4-2AT6 cell lines was 94.3 µM.

The above result showed that C4-2AT6 cell lines were enzalutamide-resistant prostate cancer cell lines having enzalutamide resistance about 7.8 times as high as that of enzalutamide non-resistant lines (LN-Cap cell lines).

### Example 6

### 6. Establishment of Cabazitaxel-resistant Prostate Cancer Cell Lines

An attempt was made to establish cabazitaxel-resistant prostate cancer cell lines on the basis of DU145 cell lines which are metastatic CRPC cell lines.

### 6-1 Method

### [Establishment of DU145CR Cell Lines]

DU145CR cell lines were established by culturing DU145 cell lines in the presence of 0.2 nM cabazitaxel (manufactured by Carbosynth Limited) for about 2 years while gradually increasing the cabazitaxel concentration to 3 nM.

### [In Vitro Analysis Using DU145CR Cell Lines]

The established DU145CR cell lines and DU145 cell lines were cultured on 96-well plates, respectively, for 24 hours, and further cultured for 24 hours in the presence of cabazitaxel at various concentrations (0 nM, 1 nM, 2nM, 4 nM, 8 nM and 16 nM), and the number of living cells was then measured with "Premix WST-1 Cell Proliferation Assay System" (manufactured by Takara Bio Inc.).

### [In Vivo Analysis Using DU145CR Cell Lines]

The 1 × 10⁶ established DU145CR cell lines and 1 × 10⁶ DU145 cell lines were subcutaneously inoculated to castrated male nude mice (BALB/C), respectively, to prepare subcutaneous tumor model mice (DU145CR mice and DU145 mice). When the tumor size reached about 200 mm³, the subcutaneous tumor model mice were divided into four groups (DU145 mouse group, cabazitaxel administration DU145 mouse group, DU145CR mouse group and cabazitaxel administration DU145CR mouse group), and to the cabazitaxel administration DU145 mouse group and the cabazitaxel administration DU145CR mouse group (n = 5 for each group), cabazitaxel (manufactured by Carbosynth Limited) was intraperitoneally administered in a single dose of 10 mg/kg (body weight). Cabazitaxel was not administered to the DU145 mouse group and the DU145CR mouse group (n = 5 for each group). Immediately after oral administration (day 0) and 4 days, 8 days and 12 days after the oral administration, tumor volumes were measured and an average value was calculated.

### 6-2 Result

### [In Vitro Analysis Using DU145CR Cell Lines]

The number of living cells in DU145CR cell lines was shown to be larger than the number of living cells in DU145 cell lines in the presence of cabazitaxel at any of the concentrations (see Figure 5A). On the basis of such a result, IC50 of cabazitaxel was measured, and resultantly found that IC50 for DU145 cell lines was 3.8 nM, whereas IC50 for DU145CR cell lines was 11.0 nM.

The above result showed that cabazitaxel-resistant prostate cancer cell lines (DU145CR cell lines) with cabazitaxel resistance increased by a factor of about 3 were established.

### [In Vivo Analysis Using DU145CR Cell Lines]

12 days after oral administration, the tumor volume in the cabazitaxel administration DU145CR mouse group was shown to be about twice the tumor volume in cabazitaxel administration DU145 mouse group (see Figure 5B).

The above result showed that cabazitaxel-resistant prostate cancer model animals with cabazitaxel resistance increased by a factor of about 2 were prepared.

### Example 7

### 7. Cell growth Suppressive Effect on Enzalutamide-resistant Prostate Cancer Cell Lines and Cabazitaxel-resistant Prostate Cancer Cell Lines

### [Method]

For confirming that the cloperastine species has a cell growth suppressive effect on enzalutamide-resistant prostate cancer and cabazitaxel-resistant prostate cancer, enzalutamide-resistant prostate cancer cell lines (C4-2AT6 cell lines) and cabazitaxel-resistant prostate cancer cell lines (DU145CR cell lines) were used to analyze the cell growth property in the presence of cloperastine species in accordance with the method described in Example 2.

### [Result]

C4-2AT6 cell lines and DU145CR cell lines cultured in the presence of cloperastine were shown to have a significantly smaller number of living cells in a concentration-dependent manner than the cell lines cultured without cloperastine (see Figure 6).

This result shows that the cloperastine species has a cell growth suppressive effect on enzalutamide-resistant prostate cancer and cabazitaxel-resistant prostate cancer.

### Example 8

### 8. Antitumor Effect on Prostate Cancer Model Mice

For confirming the cloperastine species has an antitumor effect on prostate cancer, the cloperastine species was administered to metastatic CRPC model mice and cabazitaxel-resistant prostate cancer model mice, and a change in tumor volume was analyzed.

### [Method]

1 × 10⁶ metastatic CRPC cell lines (DU145 cell lines) were subcutaneously inoculated to castrated male nude mice (BALB/C) to prepare subcutaneous tumor model mice. When the tumor size reached about 100 mm³, the subcutaneous tumor model mice (CRPC model mice) were divided into three groups (control group, cabazitaxel administration group and cloperastine administration group). To the cloperastine administration group (n = 5), a cloperastine hydrochloride (manufactured by Sigma-Aldrich Co. LLC) was orally administered daily at a dose of 12.5 mg/kg (body weight) per day in terms of cloperastine ("cloperastine administration group" in Figure 7A). To the cabazitaxel administration group (n = 5), cabazitaxel (manufactured by Carbosynth Limited) was intraperitoneally administered in a single dose of 10 mg/kg (body weight) ("cabazitaxel administration group" in Figure 7A). On the other hand, any of the cloperastine hydrochloride and cabazitaxel was not administered to the control group (n = 5) (control group in Figure 7A) .

The 1 × 10⁶ DU145CR cell lines established in Example 5 were subcutaneously inoculated to castrated male nude mice (BALB/C) to prepare subcutaneous tumor model mice (cabazitaxel-resistant CRPC mice). When the tumor size reached about 100 mm³, the subcutaneous tumor model mice were divided into three groups (control group, cabazitaxel administration group and cloperastine administration group). To the cloperastine administration group (n = 5), a cloperastine hydrochloride (manufactured by Sigma-Aldrich Co. LLC) was orally administered daily at a dose of 12.5 mg/kg (body weight) per day in terms of cloperastine ("cloperastine administration group" in Figure 7B). To the cabazitaxel administration group (n = 5), cabazitaxel (manufactured by Carbosynth Limited) was intraperitoneally administered in a single dose of 10 mg/kg (body weight) ("cabazitaxel administration group" in Figure 7B). On the other hand, any of the cloperastine hydrochloride and cabazitaxel was not administered to the control group (n = 5) ("control group" in Figure 7B). For the CRPC model mice and the cabazitaxel-resistant CRPC mice, tumor volumes were measured and an average value was calculated immediately after oral administration of the cloperastine hydrochloride (day 0) and 4 days, 8 days, 12 days and 16 days after the oral administration.

### [Result]

For the CRPC model mice, the tumor volume in the cloperastine administration group was shown to be smaller than the tumor volume in the control group (see Figure 7A). In addition, the tumor volume decrease level in the cloperastine administration group was shown to be higher than the tumor volume decrease level in the cabazitaxel administration group.

For the cabazitaxel-resistant CRPC mice, it was shown that administration of cabazitaxel hardly enabled suppression of an increase in tumor volume, whereas administration of cloperastine considerably reduced the tumor volume (see Figure 7B).

According to these results, the *in vivo* analysis showed that the cloperastine species had a high antitumor effect on CRPC, particularly cabazitaxel-resistant CRPC.

### Example 9

### 9. Cell Growth Suppressive Effect and Apoptosis Inducing Effect on Prostate Cancer Cells

For confirming that the cloperastine species has a cell growth suppressive effect and an apoptosis inducing effect on CRPC and cabazitaxel-resistant CRPC, metastatic CRPC cell lines (DU145 cell lines and PC3 cell lines) were cultured in the presence of the cloperastine species, and analysis was performed by an immunohistological staining method using an antibody to a cell growth marker (Ki67), and a TUNEL (TdT-mediateddUTPnickendlabeling) method.

### [Method]

DU145 cell lines and DU145CR cell lines were cultured on 96-well plates, respectively, for 24 hours, and to a culture solution, a cloperastine hydrochloride (manufactured by Sigma-Aldrich Co. LLC) was added to 10 µM. The cell lines were further cultured for 24 hours, and the cells were then fixed with 4% paraformaldehyde. The fixed cells were subjected to blocking treatment with a 1% BSA (bovine serum albumin) solution, and primary antibody reaction treatment with a Ki67 antibody (manufactured by Dako Company) was performed overnight at 4°C. Thereafter, secondary antibody reaction treatment with a peroxidase-labeled anti-mouse IgG antibody (manufactured by Nichirei Bioscience Inc.) was performed, and a DAB (diamino benzidine) staining method was carried out using DAB TRIS Tablets (manufactured by Muto Pure Chemicals Co., Ltd.). The immunohistological-stained cell sample was observed under a phase-contrast microscope (see Figure 8A), and the ratio of Ki67-positive cells was calculated. The above-described fixed cells were subjected to a TUNEL method using In situ Apoptosis Detection Kit (manufactured by Takara Bio Inc.). The cell sample stained by the TUNEL method was observed under a phase-contrast microscope (see Figure 9A), and the ratio of TUNEL-positive cells (cells undergoing apoptosis) was calculated.

### [Result]

DU145 cell lines and DU145CR cell lines cultured in the presence of cloperastine were shown to have a lower ratio of Ki67-positive cells (see Figure 8) and a higher ratio of TUNEL-positive cells (see Figure 9) than the cell lines cultured without cloperastine.

This result shows that the cloperastine species has a cell growth suppressive effect and an apoptosis inducing effect on CRPC having cabazitaxel resistance etc.

### Example 10

### 10. Cell Growth Suppressive Effect on Prostate Cancer Cell Lines by Use in Combination with Anticancer Agent

For confirming the effect of use of the cloperastine species in combination with an existing anticancer agent, a suppressive effect on growth of prostate cancers by use of the cloperastine species in combination with cabazitaxel was analyzed.

### [Method]

The DU145CR cell lines established in Example 5 were cultured on a 96-well plate for 24 hours, and then further cultured for 24 hours in the presence of a 5 µM or 10 µM cloperastine hydrochloride (manufactured by Sigma-Aldrich Co. LLC) and 2 nM or 4 nM cabazitaxel (manufactured by Carbosynth Limited), and the number of living cells was then measured with "Premix WST-1 Cell Proliferation Assay System" (manufactured by Takara Bio Inc.). As a control, an experiment was also conducted in which DU145CR cell lines were cultured in a culture solution free of a cloperastine hydrochloride and cabazitaxel.

### [Result]

At any of the concentrations, DU145CR cell lines cultured in the presence of cloperastine and cabazitaxel were shown to have a significantly smaller number of living cells than the cell lines cultured in the presence of cloperastine alone (see Figure 10). The combination index (CI) of cloperastine and cabazitaxel was 0.60.

These results show that use of the cloperastine species in combination with cabazitaxel produced a higher cell growth suppressive effect of prostate cancer cells than use of cloperastine species alone, and a synergetic effect was obtained by use of the cloperastine species in combination with cabazitaxel. Thus, use of the cloperastine in combination with an existing anticancer agent against prostate cancer can be highly expected to produce a higher antitumor effect while lessening side effects.

### Example 11

### 11. Antitumor Effect on Prostate Cancer Model Mice by Use in Combination with Anticancer Agent

For confirming the effect of use of the cloperastine species in combination with an existing anticancer agent, an antitumor effect on prostate cancer model mice by use of the cloperastine species in combination with cabazitaxel was analyzed.

### [Method]

The 5 × 10⁶ DU145CR cell lines established in Example 5 were subcutaneously inoculated to castrated male nude mice (BALB/C) to prepare subcutaneous tumor model mice (cabazitaxel-resistant CRPC mice). When the tumor size reached about 100 mm³, the subcutaneous tumor model mice were divided into four groups (control group, cabazitaxel administration group, cloperastine administration group and cloperastine + cabazitaxel administration group). To the cabazitaxel administration group (n = 5), cabazitaxel (manufactured by Carbosynth Limited) was intraperitoneally administered in a single dose of 10 mg/kg (body weight). To the cloperastine administration group (n = 5), a cloperastine hydrochloride (manufactured by Sigma-Aldrich Co. LLC) was orally administered daily at a dose of 12.5 mg/kg (body weight) per day in terms of cloperastine. To the cloperastine + cabazitaxel administration group (n = 5), the cloperastine hydrochloride was orally administered daily at a dose of 12.5 mg/kg (body weight) per day in terms of cloperastine, and cabazitaxel (manufactured by Carbosynth Limited) was intraperitoneally administered in a single dose of 10 mg/kg (body weight). On the other hand, any of the cloperastine hydrochloride and cabazitaxel was not administered to the control group (n = 5). For the cabazitaxel-resistant CRPC mice, tumor volumes were measured and an average value was calculated immediately after oral administration (day 0) and 4 days, 8 days and 12 days after the oral administration.

### [Result]

The tumor volume in the cloperastine + cabazitaxel administration group was significantly smaller than the tumor volume in the cloperastine administration group (see Figure 11).

This result shows that use of the cloperastine species in combination with an existing anticancer agent produces a higher antitumor effect on prostate cancer than use of the cloperastine species alone.

### Industrial Applicability

The present invention contributes to prevention or treatment of prostate cancer, particularly castration-resistant prostate cancer with cabazitaxel resistance, which is the most refractory prostate cancer.

## Claims

1. A prophylactic or therapeutic agent for prostate cancer, comprising cloperastine or a pharmaceutically acceptable salt thereof.

2. The prophylactic or therapeutic agent according to claim 1, which is orally administered.

3. The prophylactic or therapeutic agent according to claim 1 or 2, wherein the prostate cancer is castration-resistant prostate cancer or enzalutamide-resistant prostate cancer.

4. The prophylactic or therapeutic agent according to claim 3, wherein the castration-resistant prostate cancer has cabazitaxel resistance.

5. The prophylactic or therapeutic agent according to any one of claims 1 to 4, which is used in combination with an anticancer agent.

6. The prophylactic or therapeutic agent according to claim 5, wherein the anticancer agent is cabazitaxel.
